(19)

Europäisches
Patentamt
European
Patent Office
Office européen
des brevets

(11) **EP 3 513 780 A1**

(12) **EUROPEAN PATENT APPLICATION**

(43) Date of publication:
    24.07.2019  Bulletin 2019/30

(51) Int Cl.:
    *A61K 8/42* (2006.01)          *A61K 8/67* (2006.01)
    *A61Q 19/08* (2006.01)

(21) Application number: 18152954.6

(22) Date of filing: 23.01.2018

(84) Designated Contracting States:
    AL AT BE BG CH CY CZ DE DK EE ES FI FR GB
    GR HR HU IE IS IT LI LT LU LV MC MK MT NL NO
    PL PT RO RS SE SI SK SM TR
    Designated Extension States:
    BA ME
    Designated Validation States:
    MA MD TN

(71) Applicant: DSM IP Assets B.V.
    6411 TE  Heerlen (NL)

(72) Inventor: **The designation of the inventor has not
    yet been filed**

(74) Representative: **Berg, Katja**
    **DSM Nutritional Products Ltd**
    **Patent Department**
    **Wurmisweg 576**
    **4303 Kaiseraugst (CH)**

(54) **METHOD FOR SUPPORTING EPIDERMAL STEM CELL SELF-RENEWAL**

(57)    The present patent application relates to a method for inducing epidermis stem cell self-renewal as well as to
compounds or compositions for the use in epidermis stem cell self-renewal.

EP 3 513 780 A1

**Description**

The present patent application relates to a method for supporting epidermal stem cell self-renewal as well as to compounds or compositions for the use in epidermal stem cell self-renewal.

Human skin exists of three main parts: the epidermis, lying outermost, and beneath a layer of connective tissue, which includes the though collagen-rich dermis and the underlaying fatty subcutaneous layer or hypodermis. The epidermis is a multilayered (stratified) epithelium composed of a cornified layer, a granular layer, a spinous layer and a basal layer. The epidermis is separated from the dermis by a basement membrane. The cornified top layer is composed of 10 to 30 layers of polyhedral, anucleated corneocytes (final step of keratinocyte differentiation). In the granular layer, the keratinocytes lose their nuclei and their cytoplasm appears granular. In the spinous layer, the keratinocytes become connected through desmosomes. The basal layer is composed of mainly proliferating and non-proliferating keratinocytes, attached to the basement membrane by hemidesmosomes.

The epidermis is continually renewed by the production of new cells in the basal layer. Differentiating cells delaminate from the basement membrane and are displaced outwards through the epidermal layers, undergoing multiple stages of differentiation until, in the stratum corneum, losing their nucleus and fusing to squamous sheets, which are eventually shed from the surface (desquamation). Differentiated keratinocytes secrete keratin proteins which contribute to the formation of an extracellular matrix which is an integral part of the skin barrier function. In normal skin, the rate of keratinocyte production equals the rate of loss, taking about two weeks for a cell to journey from the stratum basale to the top of the stratum granulosum, and an additional two to four weeks to cross the stratum corneum. The entire epidermis is replaced by new cell growth over a period of about 28 days.

Homeostasis and therepiar capability of adult skin are maintained by the epidermal stem cells compartments. In the basal layer there have to be cells that can remain undifferentiated and carry on dividing for this whole period, continually throwing off descendants that differentiate, leave the basal layer, and are eventually discarded. The process can be maintained only if the basal cell population is self-renewing. It must therefore contain some cells that generate a mixture of progeny, including daughters that remain undifferentiated like their parent, as well as daughters that differentiate. Cells with this property are called stem cells. At steady state, to maintain a stable stem-cell population, precisely 50% of the daughters of stem cells in each cell generation must remain as stem cells.

There are many points in the process that have to be controlled according to circumstances: the rate of stem-cell division; the probability that a stem-cell daughter will remain a stem cell; the number of cell divisions of the transit amplifying cells; the timing of exit from the basal layer, and the time that the cell then takes to complete its differentiation program and be sloughed from the surface. Regulation of these steps must enable the epidermis to respond to rough usage by becoming thick and callused, and to repair itself when wounded.

Moreover, it may be stated that during life time, the rate of self-renewal becomes lower and self-renewal takes longer.

It was therefore the object of the present invention to help the epidermis to support stem cell self-renewal to develop a way of 'anti-aging' for the epidermis.

Surprisingly, it has been found that specific compounds are able to influence the stem-cell production in the epidermis. Thereby, a specific mode of action is addressed, namely the stimulation of the transcription factor p63.

Thus, the invention relates in one aspect to a method for supporting epidermis stem cell self-renewal, comprising applying an effective amount of an agent for upregulating stem cell factor p63.

Preferably in all embodiments of the present invention the agent is comprised in a topical composition intended to be applied to the skin of a human in need thereof.

The term 'an effective amount' as used herein refers to an amount necessary to obtain a physiological effect. The physiological effect may be achieved by one application dose or by repeated applications. The dosage administered may, of course, vary depending upon known factors, such as the physiological characteristics of the particular composition comprising the agent and its mode and route of administration; the age, health and weight of the recipient; the nature and extent of the symptoms; the kind of concurrent treatment; the frequency of treatment; and the effect desired and can be adjusted by a person skilled in the art.

Protein p63 (TP63) is considered a "master regulator" for epidermal homeostasis. As an essential transcription factor of the p53 family it promotes the balance between stemness, differentiation, and scenescence. p63 levels are highest in the basal layer, maintaining cell proliferation and stemness. p63 regulates epidermal homeostasis throughout the layers, contributing to barrier formation, terminal differentiation, cell adhesion, basal layer proliferation, basement membrane formation, and epidermal cell commitment. p63 expression decreases with age.

In the basal layer, p63 expression maintains stemness and proliferation. In suprabasal layers, p63 expression decreases through targeted degradation by microRNAs; the lower levels of p63 lead to reduced proliferation and enhanced differentiation and stratification.

Moreover, the role of specific micro RNAs has to be considered as regulators for p63 function and vice versa. For instance, miR-203 and miR-720 are rapidly upregulated when primary keratinocytes are induced to differentiate.

These specific microRNAs, expressed in suprabasal levels of the epidermis, target p63 for degradation. Hence, a decreased expression of miR-203 and/or miR-720 leads to an upregulation of p63, with all positive effects as described above. Therefore, the present invention also relates to the following embodiments:

(a) In one aspect of the present invention, expression of hsa-miR-720 in normal human epidermal keratinocytes (NHEK) is decreased by 1.5 to 2 times, and preferably more than 2 times, for instance 1.5 to 10 times, 1.5 to 5 times, 2 to 10 times, 2 to 5 times.

(b) In another aspect of the present invention, expression of hsa-miR-203 in normal human epidermal keratinocytes (NHEK) is decreased by 1.5 to 2 times, and preferably more than 2 times, for instance 1.5 to 10 times, 1.5 to 5 times, 2 to 10 times, 2 to 5 times.

(c) In one aspect of the present invention, expression of hsa-miR-720 and hsa-miR-203 in normal human epidermal keratinocytes (NHEK) is decreased by 1.5 to 2 times, and preferably more than 2 times, for instance 1.5 to 10 times, 1.5 to 5 times, 2 to 10 times, 2 to 5 times.

(d) In one aspect of the present invention, expression of the TP63 gene in normal human epidermal keratinocytes (NHEK) is increased by 1.1 to 1.5 times, and preferably more than 1.5 times, for instance 1.50 to 5.0 times.

(e) In one aspect of the present invention, features (a) and (d) are fulfilled, in one aspect of the invention, features (c) and (d) are fulfilled.

In a further aspect of the invention, the self-renewing capacity of basal progenitor cells is maintained. This means that the amount of basal progenitor cells remains constant, in particular that the amount of basal progenitor cells during a specific time period and within a specific area remains constant.

In a further aspect of the invention, the stemness of epidermal cells is preserved. This means that the ability of stem cells to divide and produce further stem cells in the basal layer is maintained.

In a further aspect of the invention, the epidermal stem cells damaged by factors such as UV exposure and aging are replaced. This means that further stem cells in the basal layer are produced. After differentiation, they replace those epidermal cells which have been destroyed by UV light or which are disposed from the cornified layer due to their age, at the end of their life cycle.

In a further aspect of the invention, the niche environment of epidermal stem cells and epidermal self-rejuvenation is preserved. This means that epidermal stem cells not only differentiate, but also divide in order to replace them in the niches where differentiated stem cells are leaving the layer, stem cells in stratified epithelial tissue is maintained. Molecular boundary between proliferative basal progenitors and terminally differentiating suprabasal cells are defined, ensuring proper identity of neighbouring layers.

In a further aspect of the invention, the preservation of the basal membrane is activated, which means that new basal stem cells are produced.

In a further aspect of the invention, the epidermis is self-rejuvenated, which is a result from the continued division of the stem cells in the basal layer.

In a further aspect of the invention, the present method results in a self-renewal within the epidermis, which is a result of the continued division of the stem cells in the basal layer.

In a further aspect of the invention, the normal epidermal homeostasis is maintained, which is a result of the continued division of the stem cells in the basal layer. Moreover, sufficient stem cells also contribute to the differentiation of said cells and their way out of the basal layer into upper layers.

In a further aspect of the invention, the thickness of the epidermis is increased. This results in a prevention of thinning of epidermis during the skin aging process.

In a further aspect of the invention, premature skin aging is prevented. This is because the present method activates basal stem cells in order to divide as well as in order to differentiate. This results in a self-renewal of the epidermis, thereby preventing premature skin aging.

In a further aspect of the invention, elasticity, softness and resilience of skin are increased. The self-rejuvenating action of the epidermis according to the present method leads to higher elasticity, softness and resilience of the skin.

In one embodiment of the invention, the agent of the inventive method is D-panthenol, vitamin $B_{12}$, vitamin $B_2$, pyridoxine HCl, niacinamide or folic acid, preferably it is D-panthenol.

D-panthenol is also referred to as panthenol (INCI), dexpanthenol, provitamin B5, or (+)-(R)-2,4-Dihydroxy-N-(3-hydroxypropyl)-3,3-dimethylbutyramid. D-Panthenol improves hydration, reduces itching and inflammation of the skin, and accelerates epidermal wound healing.

In particular, the agent of the inventive method such as preferably D-panthenol upregulates stem cell factor p63. The agent of the inventive method such as preferably D-panthenol further maintains self-renewing capacity of basal progenitor cells. The agent of the inventive method such as preferably D-panthenol preserves stemness of skin cells. The agent of the inventive method such as preferably D-panthenol replaces epidermal stem cells damaged by factors such as UV exposure and aging. The agent of the inventive method such as preferably D-panthenol preserves the niche environment of epidermal stem cells and epidermal self-rejuvenation. The agent of the inventive method such as preferably D-panthenol activates preservation of the basal membrane. The agent of the inventive method such as preferably D-panthenol increases the thickness of the epidermis. The agent of the inventive method such as preferably D-panthenol helps to self-rejuvenate the epidermis. The agent of the inventive method such as preferably D-panthenol maintains the normal epidermal homeostasis. The agent of the inventive method such as preferably D-panthenol prevents premature skin aging. The agent of the inventive method such as preferably D-panthenol increases elasticity, softness and resilience of skin.

Thus, the present invention also relates to the use of D-panthenol, vitamin B12, vitamin B2, pyridoxine, niacinamide, or folic acid, preferably D-panthenol, for upregulating stem cell factor p63 for:

a. maintaining the self-renewing capacity of basal progenitor cells.
b. preserving the stemness of skin cells.
c. replacing epidermal stem cells damaged by factors such as UV exposure and aging.
d. for preserving the niche environment of epidermal stem cells and epidermal self-rejuvenation.
e. activating the preservation of the basal membrane.
f. increasing the thickness of the epidermis.
g. self-rejuvenating the epidermis.
h. maintaining the normal epidermal homeostasis.
i. preventing premature aging of the epidermis.
j. increasing elasticity, softness and resilience of the epidermis.

In a further aspect of the present invention, the agent applied in the method is included in a topical composition.

Preferably, the topical composition comprises D-panthenol in an amount selected in the range of 0.001 to 10 wt.-%, preferably in the range of 0.01 to 7wt-%, more preferably in the range of 0.1 to 6 wt-% and most preferably in the range of 0.5 to 5 wt-% based on the total weight of the composition. Further preferred ranges are 0.5 to 4.5 wt-%, 0.6 to 4 wt-%, 0.6 to 3.5 wt-% D-panthenol based on the total weight of the composition.

D-panthenol is commercially available and sold for instance by DSM Nutritional Products Europe Ltd..

Vitamin $B_{12}$ is also referred to as cobalamin. Preferably, the topical composition comprises vitamin $B_{12}$ in an amount selected in the range of 0.0001 to 1 wt.-%, preferably in the range of 0.001 to 0.7wt-%, more preferably in the range of 0.01 to 0.6 wt-% and most preferably in the range of 0.05 to 0.5 wt-% based on the total weight of the composition. Further preferred ranges are 0.03 to 0.45 wt-%, 0.06 to 0.4 wt-%, 0.06 to 0.35 wt-% Vitamin $B_{12}$ based on the total weight of the composition.

Vitamin $B_2$ is also referred to as riboflavin. Preferably, the topical composition comprises vitamin $B_2$ in an amount selected in the range of 0.001 to 10 wt.-%, preferably in the range of 0.01 to 7wt-%, more preferably in the range of 0.1 to 6 wt-% and most preferably in the range of 0.5 to 5 wt-% based on the total weight of the composition. Further preferred ranges are 0.5 to 4.5 wt-%, 0.6 to 4 wt-%, 0.6 to 3.5 wt-% vitamin $B_2$ based on the total weight of the composition.

Pyridoxine HCl is also referred to as vitamin $B_6$ or pyridoxol. Preferably, the topical composition comprisespyridoxince in an amount selected in the range of 0.001 to 10 wt.-%, preferably in the range of 0.01 to 5wt-%, more preferably in the range of 0.05 to 0.5 wt-% and most preferably in the range of 0.05 to 0.3 wt-% based on the total weight of the composition. Further preferred ranges are 0.5 to 4.5 wt-%, 0.6 to 4 wt-%, 0.6 to 3.5 wt-% pyridoxine based on the total weight of the composition.

Niacinamide is also referred to as vitamin $B_3$ or nicotinamide. Preferably, the topical composition comprisesniacinamide in an amount selected in the range of 0.001 to 10 wt.-%, preferably in the range of 0.01 to 7wt-%, more preferably in the range of 0.1 to 6 wt-% and most preferably in the range of 0.5 to 5 wt-% based on the total weight of the composition. Further preferred ranges are 0.5 to 4.5 wt-%, 0.6 to 4 wt-%, 0.6 to 3.5 wt-% niacinamide based on the total weight of the composition.

Folic acid is also referred to as vitamin $B_9$. Preferably, the topical composition comprisesfolic acid in an amount selected in the range of 0.001 to 10 wt.-%, preferably in the range of 0.01 to 7wt-%, more preferably in the range of 0.1 to 1 wt-% and most preferably in the range of 0.1 to 0.2 wt-% based on the total weight of the composition. Further preferred ranges are 0.5 to 4.5 wt-%, 0.6 to 4 wt-%, 0.6 to 3.5 wt-% folic acid based on the total weight of the composition.

According to one aspect of the invention, the agent for upregulating stem cell factor p63 can be part of a topical composition.

The term "topical" is understood here to mean external application to keratinous substances, which are in particular the skin, scalp, eyelashes, eyebrows, nails, mucous membranes and hair, preferably the skin.

Preferred topical compositions in all embodiments of the present invention are emulsions containing an oily phase and an aqueous phase such as in particular an O/W, W/O, Si/W, W/Si, O/W/O, W/O/W multiple or a pickering emulsions. The amount of the oily phase (i.e. the phase containing all oils and fats) present in such emulsions is preferably at least 10 wt.-%, such as in the range of 10 to 60 wt.-%, preferably in the range of 15 to 50 wt.-%, most preferably in the range of 15 to 40 wt.-%, based on the total weight of the composition.

As the topical compositions are intended for topical application, they comprise a physiologically acceptable medium, that is to say a medium compatible with keratinous substances, such as the skin, mucous membranes, and keratinous fibres. In particular the physiologically acceptable medium is a cosmetically acceptable carrier.

The term cosmetically acceptable carrier refers to all carriers and/or excipients and/ or diluents conventionally used in cosmetic compositions.

Preferred topical compositions are skin care preparations, decorative preparations, and functional preparations.

Examples of skin care preparations are, in particular, light protective preparations, anti-ageing preparations, preparations for the treatment of photo-ageing, body oils, body lotions, body gels, treatment creams, skin protection ointments, skin powders, moisturizing gels, moisturizing sprays, face and/or body moisturizers, skin-tanning preparations (i.e. compositions for the artificial/sunless tanning and/or browning of human skin), for example self-tanning creams as well as skin lightening preparations.

Examples of decorative preparations are, in particular, lipsticks, eye shadows, mascaras, dry and moist make-up formulations, rouges and/or powders.

Examples of functional preparations are cosmetic or pharmaceutical compositions containing further active ingredients such as hormone preparations, vitamin preparations, vegetable extract preparations, anti-ageing preparations, and/or antimicrobial (antibacterial or antifungal) preparations without being limited thereto.

In a particular embodiment, the topical compositions are light-protective preparations (sun care products), such as sun protection milks, sun protection lotions, sun protection creams, sun protection oils, sun blocks or day care creams with a SPF (sun protection factor). Of particular interest are sun protection creams, sun protection lotions, sun protection milks and sun protection preparations.

The topical compositions may be in the form of a suspension or dispersion in solvents or fatty substances, or alternatively in the form of an emulsion or micro emulsion (in particular of oil-in-water (O/W-) or water-in-oil (W/O-)type, silicone-in-water (Si/W-) or water-in-silicone (W/Si-)type, PIT-emulsion, multiple emulsion (e.g. oil-in-water-in oil (O/W/O-) or water-in-oil-in-water (W/O/W-)type), pickering emulsion, hydrogel, alcoholic gel, lipogel, one- or multiphase solution or vesicular dispersion or other usual forms, which can also be applied by pens, as masks or as sprays.

The topical compositions are advantageously in the form of an oil-in-water (O/W) emulsion comprising an oily phase dispersed in an aqueous phase in the presence of an O/W emulsifier. The preparation of such O/W emulsions is well known to a person skilled in the art and illustrated in the examples.

The topical composition may additionally contain an emulsifier, for instance a phosphate ester, PEG-30 Dipolyhydroxystearate, PEG-4 Dilaurate, PEG-8 Dioleate, PEG-40 Sorbitan Peroleate, PEG-7 Glyceryl Cocoate, PEG-20 Almond Glycerides, PEG-25 Hydrogenated Castor Oil, Glyceryl Stearate (and) PEG-100 Stearate , PEG-7 Olivate, PEG-8 Oleate, PEG-8 Laurate, PEG-60 Almond Glycerides, PEG-20 Methyl Glucose Sesquistearate, PEG-40 Stearate, PEG-100 Stearate, PEG-80 Sorbitan Laurate, Steareth-2, Steareth-12, Oleth-2, Ceteth-2, Laureth-4, Oleth-10, Oleth-10/Polyoxyl 10 Oleyl Ether, Ceteth-10, Isosteareth-20, Ceteareth-20, Oleth-20, Steareth-20, Steareth-21, Ceteth-20, Isoceteth-20, Laureth-23, Steareth-100, glycerylstearatcitrate, glycerylstearate (self-emulsifying), stearic acid, salts of stearic acid, polyglyceryl-3-methylglycosedistearate. Further suitable emulsifiers are sorbitan oleate, sorbitan sesquioleate, sorbitan isostearate, sorbitan trioleate, Lauryl Glucoside, Decyl Glucoside, Sodium Stearoyl Glutamate, Sucrose Polystearate and Hydrated Polyisobuten. Furthermore, one or more synthetic polymers may be used as an emulsifier. For example, PVP eicosene copolymer, acrylates/$C_{10-30}$alkyl acrylate crosspolymer, acrylates/steareth-20 methacrylate copolymer, PEG-22/dodecyl glycol copolymer, PEG-45/dodecyl glycol copolymer, and mixtures thereof.

Another particular suitable class of O/W emulsifiers are non-ionic self-emulsifying system derived from olive oil e.g. known as (INCI Name) cetearyl olivate and sorbitan olivate (Chemical Composition: sorbitan ester and cetearyl ester of olive oil fatty acids) sold under the tradename OLIVEM 1000.

Further suitable emulsifiers are commercially available polymeric emulsifiers such as hydrophobically modified polyacrylic acid such as Acrylates/C10-30 Alkyl Acrylate Crosspolymers which are commercially available under the tradename Pemulen® TR-1 and TR-2 by Noveon.

Another class of particularly suitable emulsifiers are polyglycerol esters or diesters of fatty acids also called polyglyceryl ester/ diester (i.e. a polymer in which fatty acid(s) is/ are bound by esterification with polyglycerine), such as e.g. commercially available at Evonik as Isolan GPS [INCI Name Polyglyceryl-4 Diisostearate/Polyhydroxystearate/Sebacate (i.e. diester of a mixture of isostearic, polyhydroxystearic and sebacic acids with Polyglycerin-4)] or Dehymuls PGPH available at Cognis (INCI Polyglyceryl-2 Dipolyhydroxystearate).

Also suitable emulsifiers are polyalkylenglycolether such as Brij 72 (Polyoxyethylen(2)stearylether) or Brij 721 (Polyoxyethylene (21) Stearyl Ether e.g. available at Croda.

The at least one O/W respectively Si/W emulsifier is preferably used in an amount of 0.5 to 10 wt. % such as in particular in the range of 0.5 to 5 wt.-% such as most in particular in the range of 0.5 to 4 wt.-% based on the total weight of the composition.

Suitable W/O- or W/Si-emulsifiers are polyglyceryl-2-dipolyhydroxystearat, PEG-30 dipolyhydroxystearat, cetyl dimethicone copolyol, polyglyceryl-3 diisostearate polyglycerol esters of oleic/isostearic acid, polyglyceryl-6 hexaricinolate, polyglyceryl-4-oleate, polygylceryl-4 oleate/PEG-8 propylene glycol cocoate, magnesium stearate, sodium stearate, potassium laurate, potassium ricinoleate, sodium cocoate, sodium tallowate, potassium castorate, sodium oleate, and mixtures thereof. Further suitable W/Si-emulsifiers are Lauryl Polyglyceryl-3 Polydimethylsiloxyethyl Dimethicone and/or PEG-9 Polydimethylsiloxyethyl Dimethicone and/or Cetyl PEG/PPG-10/1 Dimethicone and/or PEG-12 Dimethicone Crosspolymer and/or PEG/PPG-18/18 Dimethicone. The at least one W/O emulsifier is preferably used in an amount of about 0.001 to 10 wt.-%, more preferably in an amount of 0.2 to 7 wt.-% with respect to the total weigh of the composition.

The topical compositions according to the present invention furthermore advantageously contain at least one co-surfactant such as e.g. selected from the group of mono- and diglycerides and/ or fatty alcohols. The co-surfactant is generally used in an amount selected in the range of 0.1 to 10 wt.-%, such as in particular in the range of 0.5 to 6 wt.-%, such as most in particular in the range of 1 to 5 wt.-%, based on the total weight of the composition. Particular suitable co-surfactants are selected from the list of alkyl alcohols such as cetyl alcohol (Lorol C16, Lanette 16), cetearyl alcohol (Lanette O), stearyl alcohol (Lanette 18), behenyl alcohol (Lanette 22), glyceryl stearate, glyceryl myristate (Estol 3650), hydrogenated coco-glycerides (Lipocire Na10) as well as mixtures thereof.

The compositions in form of O/W emulsions according to the invention can be provided, for example, in all the formulation forms for O/W emulsions, for example in the form of serum, milk or cream, and they are prepared according to the usual methods. The compositions which are subject-matters of the invention are intended for topical application and can in particular constitute a dermatological or cosmetic composition, for example intended for protecting human skin against the adverse effects of UV radiation (antiwrinkle, anti-ageing, moisturizing, anti-sun protection and the like).

In accordance with the present invention, the topical compositions may comprise further ingredients such as ingredients for skin lightening; tanning prevention; treatment of hyperpigmentation; preventing or reducing acne, wrinkles, lines, atrophy and/or inflammation; chelators and/or sequestrants; anti-cellulites and slimming (e.g. phytanic acid), firming, moisturizing and energizing, self-tanning, soothing, as well as agents to improve elasticity and skin barrier and/or further UV-filter substances and carriers and/or excipients or diluents conventionally used in topical compositions. If nothing else is stated, the excipients, additives, diluents, etc. mentioned in the following are suitable for topical compositions according to the present invention. The necessary amounts of the cosmetic and dermatological adjuvants and additives can, based on the desired product, easily be determined by the skilled person. The additional ingredients can either be added to the oily phase, the aqueous phase or separately as deemed appropriate. The mode of addition can easily be adapted by a person skilled in the art.

The cosmetically active ingredients useful herein can in some instances provide more than one benefit or operate via more than one mode of action.

The topical cosmetic compositions can also contain usual cosmetic adjuvants and additives, such as preservatives/ antioxidants, fatty substances/ oils, water, organic solvents, silicones, thickeners, softeners, emulsifiers, sunscreens, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sensates, astringents, antifoaming agents, pigments or nanopigments, e.g. those suited for providing a photoprotective effect by physically blocking out ultraviolet radiation, or any other ingredients usually formulated into cosmetic compositions. Such cosmetic ingredients commonly used in the skin care industry, which are suitable for use in the compositions are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the cosmetic and dermatological adjuvants and additives can - based on the desired product - easily be chosen by a skilled person in this field and will be illustrated in the examples, without being limited hereto.

Of course, one skilled in this art will take care to select the above mentioned optional additional compound or compounds and/or their amounts such that the advantageous properties intrinsically associated with the combination in accordance with the invention are not, or not substantially, detrimentally affected by the envisaged addition or additions.

The topical compositions in general have a pH in the range of 3 to 10, preferably a pH in the range of 4 to 8 and most preferably a pH in the range of 4 to 7. The pH can easily be adjusted as desired with suitable acids such as e.g. citric acid or bases such as NaOH according to standard methods in the art.

The topical compositions may further contain one or more emollients which soothe and soften the skin. As an example, the emollient may be dicaprylyl carbonate or C12-15alkyl benzoate. Further emollients are silicone (dimethicone, cyclomethicone), vegetable oils (grape seed, sesame seed, jojoba, etc.), butters (cocoa butter, shea butter), alcohols (stearyl alcohol, cetyl alcohol), and petrolatum derivatives (petroleum jelly, mineral oil).

The cosmetic compositions advantageously comprise preservatives or preservative booster. Preferably, the additional preservatives respectively preservative booster is selected from the group consisting of phenoxyethanol, ethylhexylglycerin, glyceryl caprylate, caprylyl glycol, 1,2-hexanediol, propanediol, propylene glycol as well as mixtures thereof. When present, the preservative respectively preservative booster is preferably used in an amount of 0.01 to 2 wt.-%, more preferably in an amount of 0.05 to 1.5 wt.-%, most preferably in an amount of 0.1 to 1.0 wt.-%, based on the total weight of the composition. It is particularly preferred, that the cosmetic compositions according to the invention does not contain any further/ other preservatives such as e.g. parabens and/ or methylisothiazolidine.

The following examples are provided to further illustrate the compositions and effects of the present invention. These examples are illustrative only and are not intended to limit the scope of the invention in any way.

**Experimental Part**

The study was conducted to understand how different vitamin B supplements influence microRNA and mRNA gene expression in keratinocytes. MicroRNAs are small, non-coding RNA molecules that function as negative regulators of gene expression. MicroRNA expression was analyzed using hsa-miR-203 and hsa-miR-720 and RNU44 as control microRNA. The current study was performed using normal human epidermal keratinocytes [NHEK].

All Test Materials were diluted in DermaLife K medium. NHEK cultured in DermaLife K medium without added supplements served as the Control group.

An initial cytotoxicity screening was performed to evaluate a range of Test Material concentrations prior to the microRNA analysis. The following non-cytotoxic test concentrations were used:

1. Vitamin $B_2$, 0.1mM
2. D-Panthenol, 0.1 mM
3. Pyridoxine HCl, 0.1 mM
4. Vitamin $B_{12}$, 0.03mM
5. Niacinamide, 0.1 mM
6. Folic Acid, 0.1mM

microRNA Expression Results:

Unpaired t-tests ($p < 0.05$, N=4) were performed to compare each Test Material group to the Control group. Statistically significant changes in expression were observed for each of the six supplements. An endogenous control microRNAs (RNU44) was used for normalization. In table 1, results are shown:

Table 1: Statistically Significant Changes in microRNA Expression of hsa-miR-203 and hsa-miR-720

| microRNA | vitamin B2 | D-panthenol | pyridoxine HCl | vitamin B12 | Niacinamide | Folic acid |
|---|---|---|---|---|---|---|
| hsa-miR-203 | ** | ** | ** | * | n.s. | * |
| | -1.96 | -1.76 | -1.78 | -1.46 | 1.03 | -1.46 |
| hsa-miR-720 | ** | *** | ** | *** | *** | ** |
| | -1.93 | -2.21 | -1.84 | -2.10 | -2.02 | -1.64 |
| *** = decrease of > 2-fold<br>** = decrease of 1.5 to 2-fold<br>* = decrease of < 1.5 fold<br>"n.s." = not statistically significant | | | | | | |

In the epidermis, p63 may directly promote the proliferation of progenitor cells and their self-renewal. miR-203, expressed in suprabasal layers, degrades p63. This leads to the suppression of proliferation after differentiation has begun. miR-203 is not expressed in the basal layer. Decreased miR-203 leads to increased p63-induced proliferation and less differentiation which was observed by a decreased expression of epidermal differentiation markers, i.e. CALML5/CLSP, KLK7, KRT1 & KRT10, LCE3D/ LEP16.

miR-203 and miR-720, expressed in suprabasal levels of the epidermis, target p63 for degradation. These microRNAs affect the autoregulatory feedback loop between p63 and iASPP (PPP1 R13L) maintaining homeostasis in the epidermal layer. miR- 574-3p and miR-720 are inhibited by iASPP. miR-203 acts through another mechanism, induced by PKC/AP1 expression; once induced, miR-203 then represses p63. miR-203 represses stemness; psoriasis keratinocytes do not show a basal phenotype. miR-203 acts as a switch regulating the exit from stemness.

TP63 epidermal regeneration/ homeostasis/ stemness

Protein 63 (TP63/ p63) is considered a "master regulator" of the epidermis. p63 levels are highest in the basal layer, maintaining cell proliferation and stemness. P63 regulates epidermal homeostasis throughout the layers, contributing to barrier formation, terminal differentiation, cell adhesion, basal layer proliferation, basement membrane formation, and epidermal cell commitment. p63 expression decreases with age. In the basal layer, p63 expression maintains stemness & proliferation. In suprabasal layers, p63 expression decreases through targeted degradation by microRNAs; the lower levels of p63 lead to reduced proliferation and enhanced differentiation and stratification.

CALML5/CLSP epidermal barrier

Calmodulin-like skin protein (CLSP) is a skin-specific, calcium-binding protein expressed primarily during keratinocyte differentiation in the lower levels of the stratum corneum. The protein is a well-established marker of late keratinocyte differentiation, directly involved in cornified envelope formation.

KLK7 epidermal barrier

Kalikrein 7 (KLK7) is involved in keratinocyte desquamation. Decreased expression points to slower differentiation/keratinocyte turnover.

KRT1 & KRT10 epidermal barrier

Keratin 1 (KRT1) and keratin 10 (KRT10) are early markers of differentiation; a dimer between KRT1 and KRT10 proteins contribute to epidermal stability. Elevated expression of KRT1 and KRT10 is seen during oxidative stress, and as terminal differentiation is induced.

LCE3D/ LEP16 epidermal barrier

The cornified cell envelope is an insoluble protein/lipid matrix that replaces the plasma membrane of terminally differentiating keratinocytes. This process is initiated by transglutaminases that cross-link proteins. Most of the genes that encode cornified envelope proteins are clustered in an Epidermal Differentiation Complex (EDC). The late cornified envelope family consists of 18 proteins. LCE3D is a component of the EDC, and plays a role in epidermal barrier formation and integrity. LCE3 proteins are expressed at an early time point during epidermal differentiation in the suprabasal layers. Late cornified envelope protein 3D (LCE3D) modulates barrier quality. Decreases in late envelope proteins can lead to a thinner barrier.

SPINK5 epidermal barrier

Serine protease inhibitor, Kazal type, 5 [SPINK5/ LEKTI] is a polyvalent protease inhibitor expressed mostly in the stratum granulosum, involved in skin barrier homeostasis. SPINK5 is a potent inhibitor of the KLK7 promoter of desquamation, and is able to inhibit corneocyte shedding.

Table 2: Gene expression by statistically significant linear fold-change data

| Gene | Linear Fold-Change Value, Each Group vs the Untreated Control | | | | | |
|---|---|---|---|---|---|---|
| | Vitamin B2 | D-Panthenol | PyridoxineHCl | Vitamin B12 | Niacinamide | Folic acid |
| TP63 | 1.34 | 1.54 | 1.55 | 1.32 | 1.21 | 1.43 |
| CALML5 | -1.24 | -1.87 | -1.90 | -1.73 | -1.71 | -1.67 |
| KLK7 | -2.03 | -1.60 | -1.44 | n.s. | n.s. | -1.41 |
| KRT1 | -4.00 | -4.68 | -4.56 | -2.42 | -2.34 | -3.27 |
| KRT10 | -2.50 | -2.26 | -2.45 | n.s. | n.s. | -1.73 |
| LCE3D | 1.60 | -1.99 | n.s. | -1.65 | -2.16 | -1.72 |
| SPINK5 | n.s. | -2.00 | -2.05 | -1.63 | n.s. | -1.79 |
| "n.s." = not statistically significant | | | | | | |

Experimental Procedures

Cell Culture, Cytotoxicity Screening: NHEK [purchased from Lifeline Cell Technologies] were cultured and maintained at 37°C with 5% $CO_2$ using DermaLife K medium according to the manufacturer's recommendations. Two days prior to treatment, NHEK were seeded at a density of -20,000 cells per $cm^2$ in 24-well plates. Cultures were approximately 75% confluent at the time of treatment.

Stock solutions and final working concentrations of each Test Material were prepared: The following treatment groups were included in the study (N=4):

1. Vitamin B2, 0.1 mM
2. D-panthenol, 0.1 mM
3. Pyridoxine HCl, 0.1 mM
4. Vitamin B12, 0.03mM
5. Niacinamide, 0.1 mM
6. Folic Acid, 0.1 mM

All Test Materials were diluted in DermaLife K medium. NHEK cultured in DermaLife K medium without added supplements served as the Control group.

Each preparation tube was mixed thoroughly by inverting 10 times and vortexing briefly.

Treatment and Maintenance of Cultures: Maintenance medium (DermaLife K) was removed from each culture well and replaced with 500 $\mu$L (for 24-well cytotoxicity screening) or 2.0 mL (for 6-well gene expression screening) prewarmed Test Medium. Following application of Test Media, the cultures were returned to the incubator at 37°C with 5 % $CO_2$ for 24 hours.

Treatment and Collection of Cell Culture for Gene Expression: Cultures were seeded at a concentration of 20,000 cells/$cm^2$ in a 6-well plate and incubated for three days at 37°C with 5 % $CO_2$. Fresh media replacements were performed every other day. On the day of treatment, maintenance medium was replaced with test medium. Following a 24 hour exposure, test medium was removed and cell lysis was performed through the addition of 700uL Qiazol lysis buffer (Qiagen) to each well. Cell lysates were immediately collected and stored at -80°C.

RNA Isolation: Total RNA was isolated from frozen cell lysates using a Qiagen miRNeasy Mini kit following the manufacturer's instructions for cultured cells. Total RNA concentration and purity were determined using a Nanodrop 2000 spectrophotometer.

cDNA synthesis: cDNA was generated from 100ng RNA using a Taqman® microRNA Reverse Transcription (RT) Kit and a Taqman® Custom RT Primer Pool, with 40 cycles of RT in an Applied Biosystems 2720 Thermal Cycler according to the manufacturer's instructions (Life Technologies).

cDNA preamplification: cDNA was preamplified using a Taqman® Custom microRNA Preamplification Primer Pool and Taqman® Preamplification Master Mix in an Applied Biosystems 2720 Thermal Cycler according to the manufacturer's instructions (Life Technologies). The preamplification reaction was run for 12 cycles.

qPCR Processing: qPCR reactions were run using validated Taqman® MicroRNA assays in a Custom OpenArray format. OpenArrays were run in a Life Technologies QuantStudio 12K Flex instrument. Each microRNA was assayed in duplicate.

Data Analysis: qPCR data was imported into RealTime StatMiner software v4.2 for statistical analysis using the relative quantitation (RQ) method. In the first step of an RQ analysis, the CT value of the target microRNA is normalized to the CT value of an endogenous control microRNA to generate the delta CT (dCT). dCT values are calculated in order to normalize for variability between the samples that may occur during the experimental procedures.

Unpaired t-tests were carried out using StatMiner software. Statistically significant changes in gene expression (p<0.05) are reported. Linear fold-change data for each subject was calculated using Microsoft Excel.

- For RQ values >1.0: Linear fold-change value = RQ value
- For RQ values <1.0: Linear fold-change value = -1/RQ value

Statistical Data Analysis Using StatMiner Software: qPCR data is typically expressed in log scale and then converted to linear fold change value. A log10RQ value of 1.0 is equivalent to a 10-fold change in gene expression. A log10RQ value of 0.3 is equivalent to a 2-fold change in gene expression. The log10RQ values have been converted to linear fold change values to simplify data interpretation.

qPCR Data Quality and Statistical Data Analysis: qPCR data quality is assessed using a combination of factors, including visual analysis of the shape of the PCR curve and the CT value. The relative amount of the gene transcript level is associated with the CT value of the PCR reaction.qPCR amplification values are an indication of the total amount of microRNA transcript present in the sample, and can impact the quality of the qPCR data. qPCR amplification takes place over a total of 40 cycles, and typically occurs before cycle 28. The relative amount of the gene transcript level is associated with the CT value of the PCR reaction.

CT values typically correspond with the following:

- CT values less than 28: associated with high transcript levels and robust, high quality qPCR data
- CT values greater than 28: lower level transcripts, less robust qPCR data; review data cautiously

The statistically significant genes in this Report had CT values less than 28.

**Experimental Procedures**

RNA isolated from the microRNA study described before was used here. cDNA synthesis: cDNA was generated from 2000ng RNA per sample using a High Capacity cDNA Synthesis Kit according to the manufacturer's instructions (Life Technologies).qPCR Processing: qPCR reactions were run using validated Taqman® gene expression assays in a Custom

OpenArray format. OpenArrays were run in a Life Technologies QuantStudio 12K Flex instrument. Each gene was assayed in duplicate.

Data Analysis: qPCR data was imported into RealTime StatMiner software v4.2 for statistical analysis using the relative quantitation (RQ) method. In the first step of an RQ analysis, the CT value of the target microRNA is normalized to the CT value of an endogenous control microRNA to generate the delta CT (dCT). dCT values are calculated in order to normalize for variability between the samples that may occur during the experimental procedures. See the Endogenous Control Selection section for more details. Unpaired t-tests were carried out using StatMiner software. Statistically significant changes in gene expression (p<0.05) are reported. Linear fold-change data for each subject was calculated using Microsoft Excel.For RQ values >1.0: Linear fold-change value = RQ value

## For RQ values <1.0: Linear fold-change value = -1/RQ value

Statistical Data Analysis Using Statminer Software: qPCR data is typically expressed in log scale and then converted to a linear fold change value. A log10RQ value of 1.0 is equivalent to a 10-fold change in gene expression. A log10RQ value of 0.3 is equivalent to a 2-fold change in gene expression. The log10RQ values have been converted to linear fold change values to simplify data interpretation.

Endogenous Control Selection

It is important to select an endogenous control gene that is consistently expressed in all of the samples in a study. Three algorithms were used to determine the most consistent endogenous control gene: Normfinder algorithm, Minimum Variance Median (minvarmed) algorithm, and the coefficient of variability (CV). Each algorithm generates a stability score for each gene, where lower stability scores reflect more consistent expression between samples. Each endogenous control gene was ranked for each algorithm (1 being the best ranking). The average ranking was calculated (for all algorithms, Table 2). Based on the shape of the amplification curves and the average rankings, HPRT1 was the most stable endogenous control gene. Statistics (t-tests) were carried out using dCT values normalized to HPRT1.

Table 2. Stability Scores and Algorithm Ranking Used for Endogenous Control Selection

Gene Normfinder Minvarmed CV Calculation Score Ranks Mean Rank

GAPDH 6.40E-02 1.36E-02 9.88E-03 4,3,3 3.333
GUSB 6.15E-02 2.92E-02 1.11 E-02 3,5,4 4.000
HPRT1 3.87E-02 7.69E-03 9.73E-03 2,2,2 2.000
PPIA 3.55E-02 1.53E-02 1.19E-02 1,4,5 3.333
UBC 6.75E-02 4.60E-03 7.92E-03 5,1,1 2.333

Bold text shows the lowest two scores for each algorithm and the lowest mean algorithm rank.

qPCR Data Quality and Statistical Data Analysis

qPCR data quality is assessed using a combination of factors, including visual analysis of the shape of the PCR curve and the CT value. The relative amount of the gene transcript level is associated with the CT value of the PCR reaction. qPCR amplification values are an indication of the total amount of gene transcript present in the sample, and can impact the quality of the qPCR data. qPCR amplification takes place over a total of 40 cycles, and typically occurs before cycle 28. The relative amount of the gene transcript level is associated with the CT value of the PCR reaction. CT values typically correspond with the following:

- CT values less than 28: associated with high transcript levels and robust, high quality qPCR data
- CT values greater than 28: lower level transcripts, less robust qPCR data; review data cautiously

All of the statistically significant genes in this Study had CT values less than 28.

**Claims**

1. A method for inducing epidermis stem cell self-renewal, comprising applying an effective amount of an agent for upregulating stem cell factor p63.

2. The method according to claim 1, wherein self-renewing capacity of basal progenitor cells is maintained.

3. The method according to claim 1 or 2, wherein the stemness of skin cells is preserved.

4. The method according to any one of claims 1 to 3, wherein epidermal stem cells damaged by factors such as UV exposure and aging are replaced.

5. The method according to any one of claims 1 to 4, wherein the niche environment of epidermal stem cells and epidermal self-rejuvenation is preserved.

6. The method according to any one of claims 1 to 5, wherein the maintenance of the basal membrane is activated.

7. The method according to any one of claims 1 to 6, wherein the thickness of the epidermis is increased.

8. The method according to any one of claims 1 to 7, wherein the epidermis is self-rejuvenated.

9. The method according to any one of claims 1 to 8, wherein the normal epidermal homeostasis is maintained.

10. The method according to any one of claims 1 to 9, wherein premature aging of the epidermis is prevented.

11. The method according to any one of claims 1 to 10, wherein elasticity, softness and resilience of the epidermis are increased.

12. The method according to any one of claims 1 to 11, wherein the agent is included in a topical composition.

13. The method according to any one of claims 1 to 12, wherein the agent is D-panthenol, vitamin $B_{12}$, vitamin $B_2$, pyridoxine, niacinamide, or folic acid, preferably D-panthenol.

14. The method according to claim 12 and 13, wherein D-panthenol is used in an amount selected in the range of 0.001 to 10 wt.-%, preferably in the range of 0.01 to 7wt-%, more preferably in the range of 0.1 to 6 wt-% and most preferably in the range of 0.5 to 5 wt-% based on the total weight of the topical composition.

15. Use of D-panthenol, vitamin B12, vitamin B2, pyridoxine, niacinamide, or folic acid, preferably D-panthenol, for upregulating stem cell factor p63.

16. Use of D-panthenol, vitamin B12, vitamin B2, pyridoxine, niacinamide, or folic acid, preferably D-panthenol according to claim 15 for maintaining the self-renewing capacity of basal progenitor cells, and/ or for preserving the stemness of skin cells and/ or for replacing epidermal stem cells damaged by factors such as UV exposure and aging and/ or for preserving the niche environment of epidermal stem cells and epidermal self-rejuvenation and/ or for activating the preservation of the basal membrane and/ or for increasing the thickness of the epidermis and/ or for self-rejuvenating the epidermis and/ or for maintaining the normal epidermal homeostasis and/ or for preventing premature skin aging of the epidermis and/ or for increasing elasticity, softness and resilience of the epidermis.

**EP 3 513 780 A1**

Europäisches Patentamt
European Patent Office
Office européen des brevets

# EUROPEAN SEARCH REPORT

Application Number

EP 18 15 2954

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2017 0099669 A (LG HOUSEHOLD & HEALTH CARE LTD [KR]) 1 September 2017 (2017-09-01) | 1-12 | INV. A61K8/42 A61K8/67 A61Q19/08 |
| Y | * paragraph [0005] - paragraph [0007] * * paragraph [0012] * * paragraph [0038] - paragraph [0039] * * paragraph [0051] * ----- | 13-16 | |
| X | KR 2016 0137079 A (LG HOUSEHOLD & HEALTH CARE LTD [KR]) 30 November 2016 (2016-11-30) | 1-12 | |
| Y | * paragraph [0088] * * paragraph [0089] - paragraph [0098] * * paragraph [0099] - paragraph [0100] * * claims 1-7 * ----- | 13-16 | |
| X | FR 2 971 159 A1 (SILAB SA [FR]) 10 August 2012 (2012-08-10) | 1-12 | |
| Y | * page 3 * * page 16 * * claims 1-12 * ----- | 13-16 | TECHNICAL FIELDS SEARCHED (IPC) |
| X | US 2014/328775 A1 (LAUGHLIN II LEO TIMOTHY [US] ET AL) 6 November 2014 (2014-11-06) * paragraph [0038] * ----- | 13-16 | A61K A61Q |
| X | WO 2015/057110 A2 (LTD LIABILITY COMPANY PHARMA GEN LLC PHARMA GEN [RU]) 23 April 2015 (2015-04-23) * page 2 - page 3 * ----- | 13-16 | |
| X | US 2005/255060 A1 (OBLONG JOHN E [US] ET AL) 17 November 2005 (2005-11-17) * paragraph [0144] - paragraph [0147] * ----- | 13-16 | |

-/--

The present search report has been drawn up for all claims

| Place of search | Date of completion of the search | Examiner |
|---|---|---|
| Munich | 2 July 2018 | Steinheimer, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

13

Europäisches
Patentamt

European
Patent Office

Office européen
des brevets

# EUROPEAN SEARCH REPORT

Application Number

EP 18 15 2954

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2011/090741 A2 (OPKO CURNA LLC [US]; COLLARD JOSEPH [US]; KHORKOVA SHERMAN OLGA [US]) 28 July 2011 (2011-07-28) * paragraph [0057] - paragraph [0059] * ----- | 13-16 | |
| Y | WO 2011/154402 A1 (CHANEL PARFUMS BEAUTE [FR]; CANDI ELEONORA [IT]; MELINO GENNARO [IT];) 15 December 2011 (2011-12-15) * the whole document * ----- | 13-16 | |
| Y | US 2016/102289 A1 (YU JUNYING [US] ET AL) 14 April 2016 (2016-04-14) * paragraph [0149] - paragraph [0151] * ----- | 13-16 | |

| TECHNICAL FIELDS SEARCHED (IPC) |
|---|

The present search report has been drawn up for all claims

| Place of search | Date of completion of the search | Examiner |
|---|---|---|
| Munich | 2 July 2018 | Steinheimer, K |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 18 15 2954

This annex lists the patent family members relating to the patent documents cited in the above-mentioned European search report.
The members are as contained in the European Patent Office EDP file on
The European Patent Office is in no way liable for these particulars which are merely given for the purpose of information.

02-07-2018

| Patent document cited in search report | | | Publication date | Patent family member(s) | | | Publication date |
|---|---|---|---|---|---|---|---|
| KR 20170099669 | A | | 01-09-2017 | NONE | | | |
| KR 20160137079 | A | | 30-11-2016 | NONE | | | |
| FR 2971159 | A1 | | 10-08-2012 | NONE | | | |
| US 2014328775 | A1 | | 06-11-2014 | CA | 2890512 | A1 | 06-11-2014 |
| | | | | CN | 104768525 | A | 08-07-2015 |
| | | | | EP | 2919748 | A1 | 23-09-2015 |
| | | | | JP | 6122134 | B2 | 26-04-2017 |
| | | | | JP | 2015537007 | A | 24-12-2015 |
| | | | | KR | 20150064751 | A | 11-06-2015 |
| | | | | US | 2014328775 | A1 | 06-11-2014 |
| | | | | WO | 2014179520 | A1 | 06-11-2014 |
| WO 2015057110 | A2 | | 23-04-2015 | RU | 2013146705 | A | 27-04-2015 |
| | | | | WO | 2015057110 | A2 | 23-04-2015 |
| US 2005255060 | A1 | | 17-11-2005 | CN | 1950066 | A | 18-04-2007 |
| | | | | EP | 1750654 | A1 | 14-02-2007 |
| | | | | US | 2005255060 | A1 | 17-11-2005 |
| | | | | WO | 2005110352 | A1 | 24-11-2005 |
| WO 2011090741 | A2 | | 28-07-2011 | CA | 2785177 | A1 | 28-07-2011 |
| | | | | CN | 102770540 | A | 07-11-2012 |
| | | | | EP | 2519634 | A2 | 07-11-2012 |
| | | | | ES | 2585829 | T3 | 10-10-2016 |
| | | | | HK | 1172369 | A1 | 27-01-2017 |
| | | | | JP | 5982288 | B2 | 31-08-2016 |
| | | | | JP | 2013515505 | A | 09-05-2013 |
| | | | | KR | 20120105023 | A | 24-09-2012 |
| | | | | RU | 2012124234 | A | 10-02-2014 |
| | | | | US | 2012295953 | A1 | 22-11-2012 |
| | | | | US | 2015126587 | A1 | 07-05-2015 |
| | | | | WO | 2011090741 | A2 | 28-07-2011 |
| WO 2011154402 | A1 | | 15-12-2011 | EP | 2580327 | A1 | 17-04-2013 |
| | | | | EP | 2711422 | A1 | 26-03-2014 |
| | | | | EP | 2711423 | A1 | 26-03-2014 |
| | | | | ES | 2459143 | T3 | 08-05-2014 |
| | | | | ES | 2526897 | T3 | 16-01-2015 |
| | | | | ES | 2529246 | T3 | 18-02-2015 |
| | | | | JP | 5886278 | B2 | 16-03-2016 |
| | | | | JP | 2013529091 | A | 18-07-2013 |
| | | | | US | 2013071370 | A1 | 21-03-2013 |
| | | | | WO | 2011154402 | A1 | 15-12-2011 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 18 15 2954

This annex lists the patent family members relating to the patent documents cited in the above-mentioned European search report.
The members are as contained in the European Patent Office EDP file on
The European Patent Office is in no way liable for these particulars which are merely given for the purpose of information.

02-07-2018

| Patent document cited in search report | Publication date | Patent family member(s) | Publication date |
|---|---|---|---|
| US 2016102289 A1 | 14-04-2016 | EP 3207122 A1<br>JP 2017532047 A<br>US 2016102289 A1<br>WO 2016061071 A1 | 23-08-2017<br>02-11-2017<br>14-04-2016<br>21-04-2016 |

EPO FORM P0459

For more details about this annex : see Official Journal of the European Patent Office, No. 12/82

page 2 of 2